# EUROPEAN PATENT APPLICATION

(11) **EP 4 067 502 A1**
(43) Date of publication of application: **05.10.2022**
(21) Application number: 20894461.1
(22) Date of filing: 24.11.2020
(51) Int. Cl.: C12Q 1/02, G01N 33/53, G01N 33/536, G01N 21/64

(54) **VISUALIZATION METHOD AND INFORMATION ACQUISITION METHOD**

(30) Priority: 27.11.2019 JP 2019214702
(71) Applicant: KONICA MINOLTA, INC., Tokyo 100-7015 (JP)
(72) Inventor: NEGISHI, Hiroshi, Tokyo, 100-7015 (JP); FURUSAWA, Naoko, Tokyo, 100-7015 (JP); NAKANO, Yasushi, Tokyo, 100-7015 (JP); GONDA, Kohsuke, Tokyo, 100-7015 (JP); KITAMURA, Narufumi, Tokyo, 100-7015 (JP); TAKANO, Mayumi, Tokyo, 100-7015 (JP)
(74) Representative: MFG Patentanwälte Meyer-Wildhagen Meggle-Freund Gerhard PartG mbB
(86) International application number: PCT/JP2020/043593
(87) International publication number: WO 2021/106840

(57) **Abstract**

The visualization method of the present invention includes the steps of: visualizing a first substance capable of binding to a target substance in a specimen; and visualizing a second substance produced by action of the first substance in the specimen.

## Description

### Technical Field

The present invention relates to a visualization method for visualizing a first substance and a second substance in a specimen, and an information acquisition method for acquiring information on a first substance and a second substance in a specimen.

### Background Art

From candidate compounds for medicines, only compounds evaluated to be effective by screening are selected. A medicine often binds to a target substance such as a receptor, an enzyme, an ion channel, and a transporter in a living body. Then, the medicine reaches a cell, binds to a target substance, and then acts on the cell to exhibit drug efficacy. Thus, the judgment on, for example, whether a medicine has reached a target substance or whether the drug that has reached the target substance exhibits drug efficacy is an important criterion in screening.

As a method for detecting a target substance or the like, an immunostaining method in which an antigen-antibody reaction or the like is used for the prepared tissue sample is known (See, for example, Non Patent Literatures 1 and 2.).

Non Patent Literature 1 describes a phase 1 test in which a PARP inhibitor ABT-888 and topotecan are used in combination. In Non Patent Literature 1, ABT-888 is orally administered, topotecan is intravenously administered, and the presence or absence of poly(ADP-ribose) (PAR) and yH2AX as a DNA damage marker is observed in a tumor and a peripheral blood mononuclear cell. The presence or absence of yH2AX is confirmed by an immunostaining method in which a fluorescent labeling substance is used.

Non Patent Literature 2 describes a relation between a DNA double-strand break by a cytotoxic drug and a DNA double-strand break associated with apoptosis. In Non Patent Literature 2, whether expression of yH2AX in a specific cell is due to a DNA double-strand break by a cytotoxic drug or due to a DNA double-strand break associated with apoptosis is analyzed by observing co-expression of multiple markers in the cell. The presence or absence of yH2AX is confirmed by an immunostaining method in which a fluorescent labeling substance is used.

### Citation List

### Non Patent Literature

Non Patent Literature 1: Shivaani Kummar et al., "Phase I Study of PARP Inhibitor ABT -888 in Combination with Topotecan in Adults with Refractory Solid Tumors and Lymphomas", Cancer Research, 2011, Volume 71, Issue 17, p. 5626-5634
Non Patent Literature 2: Angie B. Dull et al., "Development of a quantitative pharmacodynamic assay for apoptosis in fixed tumor tissue and its application in distinguishing cytotoxic drug-induced DNA double strand breaks from DNA double strand breaks associated with apoptosis", Oncotarget, 2018, Volume 9, No. 24, p. 17104-17116

### Summary of Invention

### Technical Problem

However, in Non Patent Literatures 1 and 2, because only the result of administration of a medicine or the like is evaluated, though whether or not the effect of the medicine has been obtained can be judged, the cause thereof cannot be grasped. Therefore, in the methods described in Non Patent Literatures 1 and 2, evaluation of the properties of the medicine is insufficient. For example, when absence of drug efficacy is confirmed, whether the absence is due to the medicine not reaching the target substance or whether the absence is due to another reason though the medicine has reached the target substance cannot be evaluated because the amount of the medicine that reached the target substance is unknown. In such a situation, in consideration of measures, findings on, for example, whether the dosage should be increased or whether the type of the medicine should be changed in the first place cannot be obtained, and an effective therapy and the like cannot be determined.

An object of the present invention is to provide a visualization method and an information acquisition method capable of accurately identifying a relation between a substance capable of binding to a target substance and action of the substance and accurately evaluating properties of the substance.

### Solution to Problem

The visualization method of the present invention as a means for solving the above problems includes the steps of: visualizing a first substance capable of binding to a target substance in a specimen; and visualizing a second substance produced by action of the first substance in the specimen.

The information acquisition method as a means for solving the above problems includes the steps of: obtaining a first image in which a first substance capable of binding to a target substance in a specimen is visualized; obtaining a second image in which a second substance produced by action of the first substance in the specimen is visualized; and obtaining information on the first substance and the second substance from the first image and the second image.

### Advantageous Effects of Invention

According to the present invention, a relation between a substance capable of binding to a target substance and action of the substance can be accurately identified and thus properties of the substance can be accurately evaluated.

### Brief Description of Drawings

Fig. 1 is a flowchart for describing the information acquisition method according to Embodiment 1 of the present invention.
Figs. 2A and 2B are diagrams for describing information obtained from a first image and a second image.
Figs. 3A to 3D are various immunostaining images.
Figs. 4A and 4B are graphs showing the reaching amount of the medicine and the effect of the medicine.
Fig. 5 is a flowchart for describing the visualization method according to Embodiment 2 of the present invention.

### Description of Embodiments

### [Embodiment 1]

Hereinafter, the information acquisition method according to Embodiment 1 of the present invention will be described in detail with reference to the accompanying drawings.

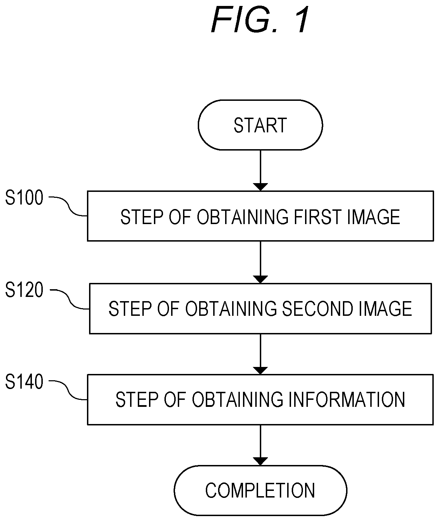

Fig. 1 is a flowchart for describing the information acquisition method according to an embodiment of the present invention.

The information acquisition method according to an embodiment of the present invention includes a step (S100) of obtaining a first image for a first substance, a step (S120) of obtaining a second image for a second substance, and a step (S140) of obtaining information on the first substance and the second substance from the first image and the second image. The step (S140) of obtaining information on the first substance and the second substance may be performed in twice after the step (S100) of obtaining a first image and after the step (S120) of obtaining a second image, or may be performed after both the steps of the step (S100) of obtaining a first image and the step (S120) of obtaining a second image are completed. The order of performing the step of obtaining a first image (S100) and the step of obtaining a second image (S120) is not particularly limited.

The "first substance" means a substance capable of binding to a target substance in a specimen. For example, the first substance is a substance administered to a specimen. The "second substance" means a substance produced by action of the first substance in a specimen. The second substance can function as a marker indicating that the first substance has acted.

The type of the specimen is not particularly limited. Examples of the specimen include blood (serum, plasma), urine, nasal fluid, saliva, feces, body cavity fluid (cerebrospinal fluid, ascites, pleural effusion and the like), biological tissues, and cells or tissues produced by culture. The type of the target substance is also not particularly limited. Examples of the target substance include nucleic acids (DNA, RNA, polynucleotide, oligonucleotide, PNA (peptide nucleic acid) or the like which may be single-stranded or double-stranded, or nucleosides, nucleotides and modified molecules thereof); proteins (polypeptides, oligopeptides, receptors present in cell membranes of target cells and the like); amino acids (including modified amino acids); carbohydrates (oligosaccharides, polysaccharides, sugar chains and the like); lipids; exosomes; and modified molecules or complexes thereof. Examples of these molecules include 5T4, AXL, BCMA, C4.4A, CA6, Cadherin3, Cadherin6, CEACAM5, CD16, CD19, CD22, CD37, CD56, CD71, CD138, CD142, CD352, DLL3, EphA2, EphrinA4, ETBR, FcyRIII, FOLR1, FGFR2, FGFR3, GCC, HER1(EGFR), HER2, HER3, IntegrinαV, LAMP1, LIV1, Mesothelin, MUC1, MUC16, NaPi2B, Nectin4, NOTCH3, PD-1, PD-L1, PSMA, PTK7, SLAMF7, SLITRK6, STEAP1, and TROP2.

In the step (S100) of obtaining a first image for a first substance, a first substance capable of binding to a target substance in a specimen is visualized to obtain a first image.

As described above, the first substance is, for example, a substance administered to a specimen. Examples of the first substance include antibodies, Antibody-Drug Conjugates (ADC), medicines such as normal pharmaceutical compounds, and toxic agents. Medicines include a substance that inhibits DNA replication in a cell. Examples of the medicines include therapeutic agents for various diseases, particularly anticancer agents.

The first substance may be one substance that is capable of binding to a target substance and produces a second substance, or may separately include a binding substance capable of binding to a target substance and an agent for producing a second substance. In the latter case, the binding substance and the agent may be bound to each other or independent of each other.

When the binding substance and the agent are bound to each other, the first substance is, for example, an antibody-drug conjugate. When the binding substance and the agent are not bound to each other, the binding substance is, for example, an antibody capable of binding to a receptor, and the agent is, for example, a drug that acts on a cell. Examples of the case where the binding substance and the agent are not bound to each other include a case in which an antibody preparation and a drug are used in combination. When the first substance is a substance that is capable of binding to a target substance and produces a second substance, the first substance is an antibody preparation. For example, when the first substance includes an antibody, an antibody-drug conjugate may be administered as the first substance, an antibody and a drug may be administered separately, or an antibody preparation may be administered.

The method for visualizing a first substance is not particularly limited. Examples of the method for visualizing a first substance include an immunostaining method and a staining method in which a molecular recognition group similar to an antibody is used.

In a method for visualizing a first substance by immunostaining method, for example, after a certain period of time has elapsed since administration of a first substance (for example, an antibody-drug conjugate), a biological tissue section (specimen) containing a target substance (for example, a receptor present in a cell membrane of a target cell) to which the first substance binds is immunostained to obtain an immunostaining image (first image) in which the first substance is visualized with a fluorescent label or an enzyme label.

In the secondary reaction of the immunostaining method, phosphor integrated particles (fluorescent dye integrated particles) can be used. The phosphor integrated particles are nano-sized particles having a structure in which particles made of an organic substance or an inorganic substance are used as a base and multiple phosphors (for example, a fluorescent dye or a semiconductor nanoparticle) are contained therein and/or adsorbed on the surface thereof. Examples of the fluorescent dye that constitutes phosphor integrated nanoparticles include rhodamine dyes, Cy dyes, Alexa Fluor (registered trademark) dyes, BODIPY dyes, squarylium dyes, cyanine dyes, aromatic ring dyes, oxazine dyes, carbopyronine dyes, and pyrromesene dyes. Examples of the material of semiconductor nanoparticles that constitute phosphor integrated nanoparticles include group II to VI semiconductors, group III to V semiconductors, and group IV semiconductors. The phosphor integrated particles can be produced according to a known method (see, for example, Japanese Patent Laid-open Publication No. 2013 - 57937).

In a method of visualizing a first substance by a staining method in which a molecular recognition group similar to an antibody is used, for example, an aptamer and a SNAP-tag are used as a molecular recognition group to obtain a first image.

When the first substance includes a binding substance and an agent, the binding substance (for example, an antibody) is preferably visualized to obtain a first image.

In the step (S120) of obtaining a second image for a second substance, the second substance produced by action of the first substance in the specimen is visualized to obtain a second image. Usually, the first image and the second image are obtained at the same point (for example, the same tissue section) or a corresponding point (for example, adjacent tissue sections) in a specimen.

As described above, the second substance is a substance produced by action of the first substance in a specimen. For example, when the first substance inhibits DNA replication in a target cell, the second substance may be a biomarker produced by inhibition of replication. The target on which the first substance acts is not particularly limited, and is, for example, a substance (for example, a protein such as an enzyme or a receptor) present in an intracellular organelle or a cell. Examples of the second substance include a DNA damage response marker, a microtubule polymerization marker, and a microtubule depolymerization marker. Examples of the DNA damage response marker include 53BP1, Ku80, MDC1, Nbs1, pATM, pATR, pChk1, pDNA-PK, Rad51, and yH2AX. Examples of the microtubule polymerization marker and the depolymerization marker include APC, CLASP, CLIP 170, EB1, EB3, MACF, MCAK, STIM1, XMAP 215, α-Tubulin, and β-Tubulin.

The method for visualizing a second substance is not particularly limited. Examples of the method for visualizing a second substance include an immunostaining method and a staining method in which a molecular recognition group similar to an antibody is used.

In a method for visualizing a second substance by immunostaining method, for example, after a certain period of time has elapsed since administration of a first substance (for example, an antibody-drug conjugate), a biological tissue section (specimen) containing a second substance (for example, a DNA fragment) produced by action of the first substance is immunostained to obtain an immunostaining image (second image) in which the second substance produced by action of the first substance is visualized with a fluorescent label or an enzyme label.

In a method of visualizing a second substance by a staining method in which a molecular recognition group similar to an antibody is used, for example, an aptamer and a SNAP-tag are used as a molecular recognition group to obtain a second image.

When the first substance includes a binding substance and an agent, a second substance (for example, a DNA fragment) produced by not the binding substance (for example, an antibody) but the agent (for example, a drug) is preferably visualized to obtain a second image.

In the step (S140) of obtaining information on the first substance and the second substance from the first image and the second image, information on the first substance and the second substance is obtained from the first image and the second image.

The type of information on the first substance and the second substance is not particularly limited as long as the information is obtained from the first image and the second image. The information on the first substance and the second substance includes information on the position or amount of the first substance and information on the position or amount of the second substance.

For example, in the step of obtaining information, the amount of the binding substance (for example, an antibody, an antibody component of an antibody-drug conjugate) bound to the target substance and the amount of the second substance produced by action of the first substance (for example, a drug component of an antibody drug conjugate) can be obtained. Information on action or effect of the first substance can be further obtained based on the obtained information on the first substance and the second substance. For example, as the information on action or effect of the first substance, information on the relation between the position or amount of the binding substance bound to the target substance and the position or amount of the second substance produced by action of the first substance may be further produced. Specifically, the information includes information on how much the second substance is produced relative to the amount of the first substance or the binding substance bound to the target substance, and information on how many cells have produced the second substance relative to the amount of the first substance or the binding substance bound to the target substance. In some cases, the binding substance that has reached the target cell may be regarded as a binding substance bound to the target substance.

For example, when the first substance includes a binding substance and an agent that are bound to each other (for example, when the first substance is an antibody-drug conjugate), the position or amount of the first substance (for example, an antibody-drug conjugate) bound to the target substance can be obtained from the first image. From the second image, the position or amount of the second substance produced by action of the first substance (for example, the drug component of the antibody-drug conjugate) can be obtained.

When the first substance includes a binding substance (for example, an antibody) and an agent (for example, a drug) that are independent of each other, the position or amount of the binding substance (for example, an antibody) bound to the target substance can be obtained from the first image. From the second image, the position or amount of the second substance produced by action of the agent (for example, drug) can be obtained.

Furthermore, when the first substance is a substance that is capable of binding to a target substance and produces a second substance, the position or amount of the first substance (for example, an antibody preparation) bound to the target substance can be obtained from the first image. From the second image, the position or amount of the second substance produced by action of the first substance (for example, an antibody preparation) can be obtained.

Therefore, information on the first substance and the second substance can be obtained from the first image and the second image, and information on action or effect of the first substance can also be obtained as necessary. For example, as information on action or effect of the first substance, information on, for example, the amount of the first substance (for example, antibody-drug conjugate and antibody preparation) that has reached the target substance, and the number, position, and type of cells that have produced the second substance by action of the first substance (for example, antibody-drug conjugate and antibody preparation) can be obtained.

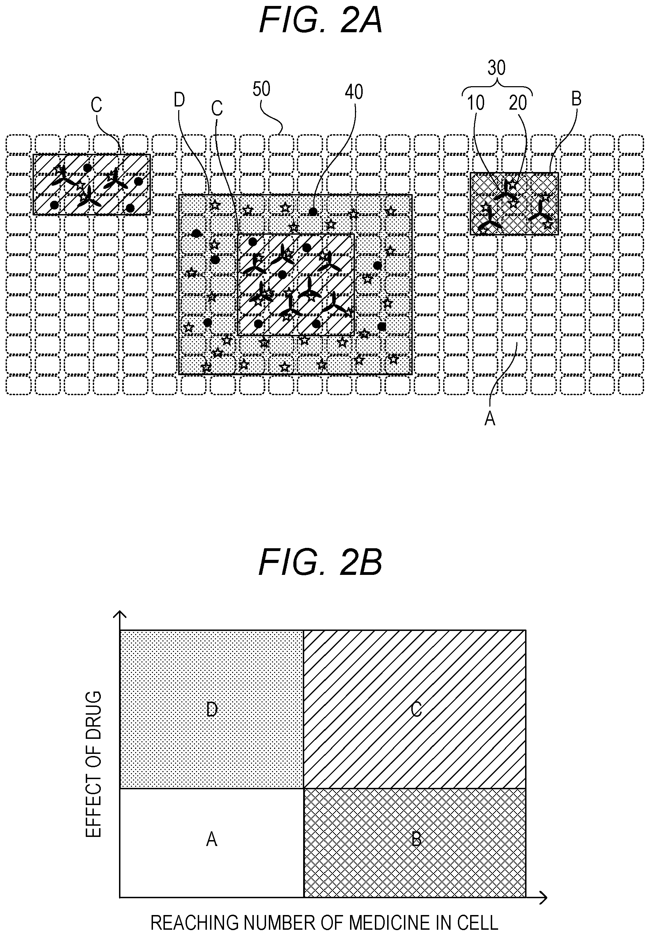

Information obtained from the first image and the second image will be specifically described. Figs. 2A and 2B are diagrams for describing information obtained from a first image and a second image. Fig. 2A is a schematic view of a biological tissue section after administration of a medicine, showing an image in which a first image and a second image are superimposed. Fig. 2B is a schematic view showing an example of a graph summarizing information obtained from Fig. 2A. The horizontal axis in Fig. 2B represents the reaching number of the first substance (for example, a medicine) in one cell, and the vertical axis represents the amount of the second substance (for example, the effect of a drug) in one cell.

Here, an antibody-drug conjugate 30 in which an antibody 10 and a drug 20 (open star) are bound is assumed to be administered as the first substance. As shown in Fig. 2A, upon administration of the antibody-drug conjugate 30, the antibody-drug conjugate 30 moves to a receptor (not shown) present in the cell membrane of a target cell 50. The antibody-drug conjugate 30 that has moved to the receptor binds to the receptor. When the antibody-drug conjugate 30 binds to the receptor, the drug 20 acts to produce the second substance 40 (solid circle).

For example, information obtained from the first image and the second image is evaluated in a unit of regions where the drug 20 acts. For example, when the drug 20 acts in cytoplasm, the evaluation may be performed in a cell unit, and when the drug 20 acts in a cell nucleus, the evaluation may be performed in a cell nucleus unit. Evaluation may be performed in a unit of the prepared biological tissue sections. In the examples shown in Figs. 2A and 2B, evaluation is performed in a cell unit.

Such evaluation can be performed by image analysis in which the following processes are performed on the first image and the second image using, for example, image processing software (Imaris; Bitplane). Here, for description, the first image is assumed to be a fluorescent image obtained by observing the first substance by fluorescent immunostaining, and the second image is assumed to be a fluorescent image obtained by observing the second substance by fluorescent immunostaining. Also for description, an image obtained by staining DNA in a cell nucleus with the fluorescent dye DAPI and an image obtained by staining a cell membrane with another fluorescent dye are also assumed to have been obtained from a biological tissue section same as or corresponding to the first image and the second image. However, staining of the cell nucleus and staining of the cell membrane may be omitted as necessary. The following processing is an example processing of image analysis, and is not limited thereto.

### (1) Image Preprocessing

The image preprocessing may not be performed, and is performed as necessary for improving the accuracy of analysis when the image quality of the first image and/or the second image is poor. Specifically, by removing noise from the imaging element of the microscopic image to repair the shape of the cell nucleus, repair the cut portion of the cell membrane, or repair the shape of the bright spot object, the accuracy of the subsequent processing can be improved.

### (2) Determination of region of cell nucleus (cell nucleus segmentation)

With respect to the staining images of the cell nuclei corresponding to the first image and the second image, the optimal diameter of the cell nuclei is set within a range of 6 to 12 µm while visually observing images. Then, a site where the center intensity ratio of DAPI (the ratio of image signal values of the center to the surrounds) is 5 or more is set as the center position (Seed Point) of the cell nucleus. The region of the cell nucleus is determined within a range of the fluorescence intensity (image signal value) of 50 to 200 while visually observing images.

The region of the cell nucleus may be determined only by the information of the region of the cell nucleus. The optimal diameter of the cell nucleus, the center position of the cell nucleus, and the region of the cell nucleus may be determined based on the information obtained by the deep learning.

### (3) Compartmentalization of cell area

With respect to the staining images of the cell membrane corresponding to the first image and the second image, the optimum minimum diameter of the cell is set within a range of 6 to 20 µm to determine the cell area while visually observing images. Then, the positional information of the cell nucleus obtained in the (2) above is input to each of the obtained cell region. When one cell region contains one cell nucleus, the region is regarded as a cell. On the other hand, when one cell region contains two or more cell nuclei, the cell region is divided to contain one cell nucleus. When one cell region contains no cell nucleus, the cell region is not regarded as a cell.

The minimum diameter of the cell may be determined based on the information obtained by deep learning. When the evaluation unit is not a cell, this processing may be omitted.

### (4) Measurement of Amount of First Substance

For the first image, the optimum diameter of the bright spot (Foci) (range where there is a clear difference in fluorescence intensity from the background) is determined within a range of 0.5 to 2 µm while visually observing the image. Then, a site where the center intensity ratio of fluorescent substance (the ratio of image signal values of the center to the surrounds) is 10 or more is set as the center position of the bright spot. The optimum region of the bright spot is determined within a range of the fluorescence intensity of fluorescence emitted from the fluorescent substance (image signal value) of 50 to 200 while visually observing the image. The sum (integrated value) of the fluorescence intensities corresponding to the bright spot included in the obtained bright spot region of the fluorescent substance is calculated. Thereafter, the calculated integrated value of the fluorescence intensity may be used as the amount of the first substance as it is, or the calculated integrated value of the fluorescence intensity may be separately converted into the amount of the first substance.

### (5) Measurement of Amount of Second Substance

For the second image, the optimum diameter of the bright spot (range where there is a clear difference in fluorescence intensity from the background) is determined within a range of 0.5 to 2 µm while visually observing the image. The optimum center position of the bright spot is determined within a range of the center intensity ratio of the fluorescent substance (the ratio of image signal values of the center to the surrounds) of 10 to 200 while visually observing the image. The optimum region of the bright spot is determined within a range of 50 to 200 while visually observing the image. The sum (integrated value) of the fluorescence intensities corresponding to the bright spot included in the obtained bright spot region of the fluorescent substance is calculated. Thereafter, the calculated integrated value of the fluorescence intensity may be used as the amount of the second substance as it is, or the calculated integrated value of the fluorescence intensity may be separately converted into the amount of the second substance.

The diameter of the bright spot, the center position of the bright spot, and the region of the bright spot may be determined based on the information obtained by the deep learning. The sum (integrated value) of the fluorescence intensities corresponding to the bright spot included in the cell nucleus region may be also used.

### (6) Output Of Analysis Result

For the first image and the second image (or superimposed image thereof), cell identification numbers are assigned to individual cell areas. Then, for the first image (or superimposed image), the sum of fluorescence intensities corresponding to the bright spot indicating the first substance whose center position is included in each cell area to which the cell identification number is assigned is calculated. Then, for the second image (or superimposed image), the sum of fluorescence intensities corresponding to the bright spot indicating the second substance whose center position is included in each cell area to which the cell identification number is assigned is calculated.

Then, the data is plotted on coordinates in which the horizontal axis represents the amount of the first substance that has reached the cell and the vertical axis represents the amount of the second substance. The effect of the first substance may be evaluated by an average or a median value of each parameter. For example, if the average of the fluorescence intensity indicating the first substance is 10 and the average of the fluorescence intensity indicating the second substance is 20 when a drug A is administered as the first substance, and the average of the fluorescence intensity indicating the first substance is 30 and the average of the fluorescence intensity indicating the second substance is 20 when a drug B is administered as the first substance, the drug A can be presumed to exert the effect with a smaller amount.

Hereinafter, with reference to Figs. 2A and 2B, an example of plotting data on coordinates in which the horizontal axis represents the amount of the first substance that has reached cells and the vertical axis represents the amount of the second substance will be described.

As shown in Figs. 2A and 2B, in the region A where the first substance 30 and the second substance 40 are not observed, it is considered that the first substance 30 has not reached, thus the intended effect has not been exhibited, and the second substance 40 has not been produced. On the other hand, in the region B where the first substance 30 is observed but the second substance 40 is not observed, it is considered that the first substance has not been able to exhibit the intended effect, for example, because the cell has resistance to the first substance (antibody-drug conjugate) 30. In the region C where the first substance 30 and the second substance 40 are observed, it is considered that the intended effect is exhibited by the first substance (antibody-drug conjugate) 30. In the region D where the first substance 30 is not observed but the second substance 40 is observed, it is considered that the effect is also exerted on cells around the cell to which the first substance (antibody-drug conjugate) 30 has reached, and the bystander effect is obtained.

In the examples shown in Figs. 2A and 2B, the case where the first substance includes a binding substance and an agent that are bound to each other has been described. However, even when the first substance includes a binding substance (for example, an antibody) and an agent (for example, a drug) that are independent of each other, or when the first substance is a substance that is capable of binding to a target substance and produces the second substance, the action or effect of the first substance can be also evaluated by the same method.

### (Effects)

As described above, in the information acquisition method according to the present embodiment, since information on the first substance and the second substance is obtained from the first image in which the first substance is visualized and the second image in which the second substance is visualized, both information on the first substance and information on the second substance reflecting the action of the first substance can be grasped, and the relation between the first substance and the action of the first substance can be accurately identified. This makes it possible to accurately evaluate the properties of the first substance. For example, when the first substance is a medicine including an antibody and a drug and the second substance is a substance produced by action of the medicine on a cell, the relation between the medicine and the action of the medicine can be accurately identified, and the properties of the medicine can be accurately evaluated.

### [Embodiment 2]

Then, the visualization method according to Embodiment 2 of the present invention will be described.

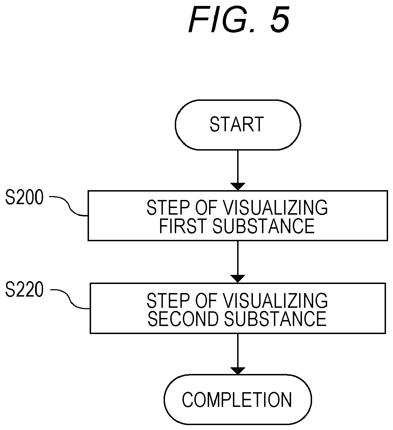

Fig. 5 is a flowchart for describing the visualization method according to Embodiment 2 of the present invention.

The visualization method according to Embodiment 2 of the present invention includes a step of visualizing a first substance (S200) and a step of visualizing a second substance (S220). The order of performing the step of visualizing a first substance (S200) and the step of visualizing a second substance (S220) is not particularly limited. The step of visualizing a first substance (S200) and the step of visualizing a second substance (S220) can be performed simultaneously.

In the step of visualizing a first substance (S200), a first substance capable of binding to a target substance in a specimen is visualized. The step of visualizing a first substance (S200) can be performed by the same method as the "method of visualizing a first substance" in Embodiment 1. The specimen, the target substance, and the first substance are the same as those in Embodiment 1.

In the step of visualizing a second substance (S220), the second substance produced by action of the first substance in the specimen is visualized. The step of visualizing a second substance (S220) can be performed by the same method as the "method of visualizing a second substance" in Embodiment 1. The second substance is the same as those in Embodiment 1.

### (Effects)

As described above, in the visualization method according to the present embodiment, the relation between the first substance and the second substance can be accurately identified.

### Examples

Hereinafter, the present invention will be described in more detail with reference to Examples, but the present invention is not limited to these Examples.

In this Example, an experiment for combination therapy of trastuzumab, an anti-HER2 humanized monoclonal antibody, and irinotecan, an anti-malignant tumor agent (type I topoisomerase inhibitor) will be shown below. In this Example, trastuzumab and irinotecan correspond to the first substance, and yH2AX, which is a marker of DNA damage, corresponds to the second substance.

### [Preparation of Sample]

### (Production of Tissue Section)

A CDX model mouse was produced by transplanting a cell mass of human breast cancer cultured cell line KPL-4 into an acquired immunodeficient mouse. Specifically, a cell mass of KPL-4 was transplanted subcutaneously into an acquired immunodeficient mouse. When the tumor tissue grew to about 800 mm³ (after 1 month from transplantation), Herceptin (registered trademark) was administered to the mouse so as to be 15 mg/kg as trastuzumab. Then, 20 hours after administration of trastuzumab, irinotecan was administered to the mouse so as to be 10 mg/kg. Four hours after irinotecan was administered, the tumor tissue was excised from the mouse, and an FFPE (formalin-fixed paraffin embedded) tissue section was prepared and placed on a slide glass. For comparison, an FFPE tissue section of a tumor tissue excised from an acquired immunodeficient mouse transplanted with a cell mass of KPL-4 without administration of trastuzumab and irinotecan was also prepared.

### (Pretreatment of Section)

The produced sections were deparaffinized, and the paraffin was replaced with pure water. After washing the sections, the sections were heated in a 10 mM citrate buffer (pH 6.0) at 121°C for 5 minutes to activate the antigen. The section after the activation treatment was washed with PBS. The obtained sections were blocked for 15 minutes using a diluent for fluorescent nanoparticles [(a solution containing 1% of casein (composition = α-casein: 50 w/w%, β-casein BSA: 50 w/w%) and BSA].

### (Primary Reaction of Fluorescent Immunostaining)

A primary reaction treatment liquid was prepared by each diluting biotin-modified anti-human IgG antibody, anti-γH2AX rabbit monoclonal antibody (EP 854 (2) Y; Abcam), anti-ATPase mouse monoclonal antibody (464.6; Abcam) to a concentration of 2 µg/mL to form a cocktail using a diluent for fluorescent nanoparticles. The sections after the blocking treatment were immersed in the primary reaction treatment liquid, and reacted overnight at 4°C. The biotin-modified anti-human IgG antibody was prepared by biotin-labeling an anti-human IgG antibody (Abcam, product number: ab 97161) using a biotin-labeling kit (Biotin Labeling Kit-NH2; DOJINDO LABORATORIES).

### (Secondary Reaction of Fluorescent Immunostaining)

A first secondary reaction treatment liquid was prepared by each diluting anti-rabbit IgG goat oligoclonal antibody labeled with the fluorescent dye Alexa 647 (Thermo Fisher Scientific) and an anti-mouse IgG goat oligoclonal antibody labeled with the fluorescent dye Alexa 488 (Thermo Fisher Scientific) to 2 µg/mL with a diluent for fluorescent nanoparticles to form a cocktail. The sections after the primary reaction treatment was washed with PBS, then immersed in this first secondary reaction treatment liquid, and reacted at room temperature for 30 minutes.

A second secondary reaction treatment liquid was prepared by diluting streptavidin-modified Texas Red dye-containing melamine resin particles (hereinafter, also referred to as fluorescent dye integrated particles) to 0.02 nM with a diluent for fluorescent nanoparticles. The sections after the reaction with the first secondary reaction treatment liquid was immersed in the second secondary reaction treatment liquid, and reacted at room temperature for 2 hours.

In 22.5 mL of pure water, 2.5 mg of a Texas Red (registered trademark) dye (Sulforhodamine 101:; excitation wavelength: 595 nm, emission wavelength: 615 nm; Sigma-Aldrich) was dissolved, and then fluorescent dye integrated particles were stirred with a hot stirrer for 20 minutes while maintaining the temperature of the solution at 70°C. To the solution after stirring, 1.5 g of a melamine resin "Nikalac MX-035" (manufactured by NIPPON CARBIDE INDUSTRIES CO., INC.) was added, and the mixture was further heated and stirred under the same conditions for 5 minutes. Formic acid (100 µL) was added to the solution after stirring, the mixture was stirred for 20 minutes while maintaining the temperature of the solution at 60 °C, and then the solution was allowed to stand and cooled to room temperature. The solution after cooling was dispensed into multiple centrifuge tubes, and centrifuged at 12,000 rpm for 20 minutes to precipitate fluorescent dye integrated particles contained in the solution. The supernatant was removed, and the precipitated fluorescent dye integrated particles were washed with ethanol and water. One thousand of the obtained fluorescent dye integrated particles were observed with a SEM, and the average particle size was measured and found to be 80 nm.

The fluorescent dye integrated particles (0.1 mg) were dispersed in 1.5 mL of ethanol, aminopropyltrimethoxysilane (LS-3150; Shin-Etsu Chemical Co., Ltd.) (2 µL) was added, and the mixture was reacted for 8 hours to perform surface amination treatment. Then, the particles subjected to the surface amination treatment were adjusted to 3 nM using PBS (phosphate buffer saline) containing 2 mM EDTA (ethylenediaminetetraacetic acid), SM (PEG) 12 (succinimidyl-[(N-maleimidopropionamido)-dodecaethyleneglycol] ester; Thermo Scientific) was mixed with this solution to a final concentration of 10 mM, and the mixture was reacted for 1 hour. This mixed solution was centrifuged at 10,000 G for 20 minutes, the supernatant was removed, then PBS containing 2 mM EDTA was added, the precipitate was dispersed, and centrifugation was performed again. Washing by the same procedure was performed three times to obtain maleimide-modified fluorescent dye integrated particles. On the other hand, streptavidin (Wako Pure Chemical Industries, Ltd.) was subjected to a thiol group addition treatment using N-succinimidyl S-acetylthioacetate (SATA), and then filtered through a gel filtration column to obtain a thiol group-added streptavidin solution capable of binding to the maleimide-modified fluorescent dye integrated particles. Maleimide-modified fluorescent dye integrated particles and thiol group-added streptavidin were mixed in PBS containing 2 mM EDTA, and reacted at room temperature for 1 hour. Then, 10 mM mercaptoethanol was added thereto to stop the reaction. The obtained mixed liquid was concentrated with a centrifugal filter, and then unreacted streptavidin and the like were removed using a gel filtration column for purification to obtain streptavidin-modified fluorescent dye integrated particles.

### (Staining of Cell Nucleus)

A cell nucleus staining treatment liquid was prepared by diluting DAPI (Sigma), a fluorescent dye for cell nucleus staining, with ultrapure water to 10 µg/mL. The sections after the secondary reaction were washed with PBS, then immersed in a cell nucleus staining treatment liquid, and reacted at room temperature for 15 minutes.

### (Aftertreatment of Section)

After the immunostained sections were washed with pure water for 10 minutes, the procedure of immersing the sections in pure ethanol was repeated 4 times to perform fixation and dehydration treatment. Then, the procedure of immersing the sections in xylene was repeated three times to perform a clearing treatment. Then a mounting agent (Marinol; MUTO PURE CHEMICALS CO., LTD.) was placed and a cover glass was placed for sealing treatment to prepare a sample to be used for observation.

### [Evaluation]

### (Confocal Microscope Observation)

For fluorescence observation of the sample, a confocal laser microscope (AIR +; Nikon Corporation) was used. The objective lens used was 60 x (Oil, ne = 1.40), and the immersion oil used had a refractive index of 1.51 (Nikon Corporation). A laser (output: 10%) having a wavelength of 405 nm was used as excitation light for observation of DAPI in which cell nuclei are stained, a laser (output: 1%) having a wavelength of 488 nm was used as excitation light for observation of ATPase-Alexa 488 in which cell membranes are stained, a laser (power: 0.5%) with a wavelength of 561 nm was used as excitation light for observation of the Texas Red integrated melamine particles stained with trastuzumab, and a laser (output: 2%) having a wavelength of 640 nm was used as excitation light for observation of yH2AX-Alexa 647 that exhibits drug efficacy. The detector sensitivity (HV) at the time of photographing was adjusted to a range in which the luminance of the image was not saturated.

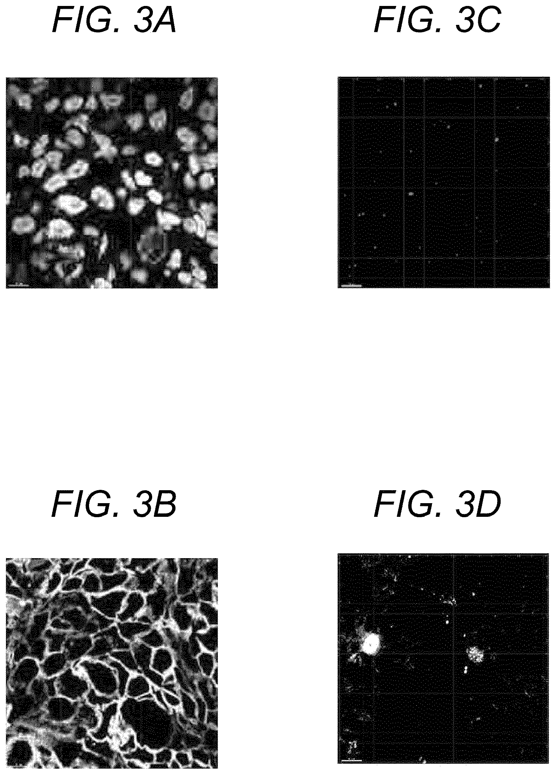

Figs. 3A to 3D are four types of fluorescence images for the same region of a section. Fig. 3A is a fluorescence image of DAPI showing a cell nucleus, Fig. 3B is a fluorescence image of ATPase-Alexa 488 showing a cell membrane, Fig. 3C is a fluorescence image (first image) of Texas Red integrated melamine particles showing trastuzumab, and Fig. 3D is a fluorescence image (second image) of yH2AX-Alexa 647 showing drug efficacy.

### (Image Analysis)

For image analysis, image processing software (Imaris; Bitplane) was used.

### (1) Determination of region of cell nucleus

The fluorescence image of DAPI shown in Fig. 3A was input as a Source Channel. Then, the optimum diameter of the cell nucleus was set within a range of 6 to 12 µm while visually observing the image. Then, a position where the center intensity ratio of DAPI (the ratio of image signal values of the center to the surrounds) was 5 or more was set as the center position (Seed Point) of the cell nucleus. The region of the cell nucleus was determined within a range of the fluorescence intensity (image signal value) of DAPI of 50 to 200 while visually observing the image.

### (2) Compartmentalization of Cell Area

The fluorescence image of Alexa 488 shown in Fig. 3B was input as a Source Channel. Then, the optimum minimum diameter of the cell was set within a range of 6 to 20 µm while visually observing the image. Then, the positional information of the cell nucleus obtained in the (1) above was input to the obtained cell region. When one cell region contains one cell nucleus, the region was regarded as a cell. On the other hand, when one cell region contains two or more cell nuclei, the cell region was divided to contain one cell nucleus. When a cell region contains no cell nucleus, the cell region was not regarded as a cell.

### (3) Measurement of amount of trastuzumab

The fluorescence image of Texas Red integrated melamine particles shown in Fig 3C was input as a Source Channel. Then, the optimum diameter of the bright spot (Foci) (range where there is a clear difference in fluorescence intensity from the background) was determined within a range of 0.5 to 2 µm while visually observing the image. Then, a position where the center intensity ratio of the Texas Red integrated melamine particles (the ratio of image signal values of the center to the surrounds) was 10 or more was set as the center position of the bright spot. The optimum region of the bright spot was determined within a range of the fluorescence intensity of the Texas Red integrated melamine particles (image signal value) of 50 to 200 while visually observing the image. The sum (integrated value) of fluorescence intensities corresponding to bright spots included in the bright spot region of the obtained Texas Red integrated melamine particles was calculated and taken as the amount of trastuzumab.

### (4) Measurement of amount of yH2AX

The fluorescence image of Alexa 647 shown in Fig. 3D was input as a Source Channel. The optimum diameter of the bright spot (range where there is a clear difference in fluorescence intensity from the background) was determined within a range of 0.5 to 2 µm while visually observing the image. The optimum center position of the bright spot was determined within a range of the center intensity ratio of Alexa647 (the ratio of image signal values of the center to the surrounds) of 10 to 200 while visually observing the image. The optimum region of the bright spot was determined within a range of 50 to 200 while visually observing the image. The sum (integrated value) of the fluorescence intensities corresponding to the bright spot included in the obtained bright spot region of Alexa647 was calculated and taken as the amount of yH2AX.

### (5) Output of Analysis Result

The cell areas were assigned cell identification numbers. Then, the sum of fluorescence intensities of the bright spot of Texas Red integrated melamine particles including a center position in each cell area assigned with a cell identification number was calculated. The sum of fluorescence intensities of the bright spot of Alexa647 including a center position in each cell area assigned with a cell identification number was calculated.

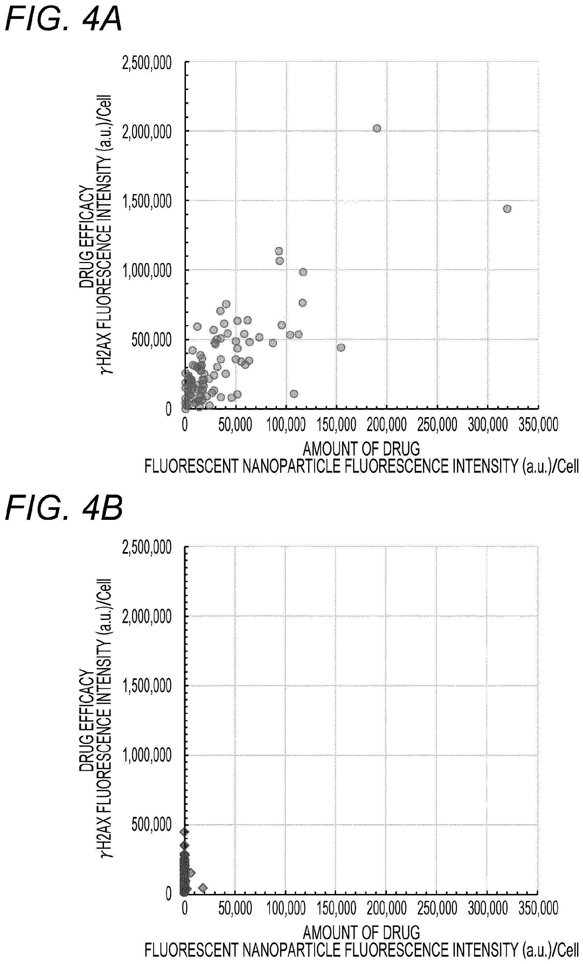

Finally, the calculation results for each cell were plotted on the coordinates. Fig. 4A is a graph showing the reaching amount of the first substance (trastuzumab) and the effect of the first substance (irinotecan) when the first substance (trastuzumab and irinotecan) is administered, and Fig. 4B is a graph showing the reaching amount of the first substance and the effect of the first substance in a group in which the first substance was not administered. The horizontal axes in Figs. 4A and 4B indicate the amount of trastuzumab that reached cells, and the vertical axes indicate the amount of yH2AX. This makes it possible to visualize how many cells are contained in each region shown in Fig. 2B.

The present application claims priority based on Japanese Patent Application No. 2019-214702 filed on November 27, 2019. The contents described in the specification and drawings of this application are all incorporated herein by reference.

### Industrial Applicability

According to the present invention, the present invention is useful, for example, for screening a medicine, proving a mechanism of action of a medicine, and evaluating toxicity of a medicine.

### Reference Signs List

- 10: Antibody
- 20: Drug
- 30: Antibody-Drug Conjugate
- 40: Second Substance
- 50: Target Cell

## Claims

1. A visualization method, comprising the steps of:
visualizing a first substance capable of binding to a target substance in a specimen; and
visualizing a second substance produced by action of the first substance in the specimen.

2. The visualization method according to claim 1, wherein in the step of visualizing a first substance, the first substance is visualized using fluorescent dye integrated particles.

3. The visualization method according to claim 1 or 2, wherein the first substance includes a binding substance capable of binding to the target substance and an agent for producing the second substance.

4. The visualization method according to claim 3, wherein the binding substance and the agent are bound to each other.

5. The visualization method according to claim 3, wherein the binding substance and the agent are independent of each other.

6. The visualization method according to claim 1 or 2, wherein the first substance is capable of binding to the target substance and produces a second substance.

7. The visualization method according to any one of claims 3 to 5, wherein the target substance is a receptor present in a cell membrane of a target cell,
the binding substance is an antibody capable of binding to the receptor,
the agent is a medicine that acts on a cell, and
the second substance is a substance produced by action of the medicine on the cell.

8. The visualization method according to claim 7, wherein the medicine includes a substance that inhibits DNA replication in the cell, and
the second substance includes a biomarker produced by inhibition of DNA replication in the cell.

9. An information acquisition method, comprising the steps of:
obtaining a first image in which a first substance capable of binding to a target substance in a specimen is visualized;
obtaining a second image in which a second substance produced by action of the first substance in the specimen is visualized; and
obtaining information on the first substance and the second substance from the first image and the second image.

10. The information acquisition method according to claim 9, wherein the first substance includes
a binding substance capable of binding to the target substance; and
an agent for producing the second substance; and
in the step of obtaining a first image, an image in which the binding substance is visualized is obtained,
and in the step of obtaining a second image, an image in which the second substance produced by action of the agent is visualized is obtained.

11. The information acquisition method according to claim 10, wherein the binding substance and the agent are bound to each other.

12. The information acquisition method according to claim 10, wherein the binding substance and the agent are independent of each other.

13. The information acquisition method according to claim 9, wherein the first substance is capable of binding to the target substance and produces a second substance.

14. The information acquisition method according to any one of claims 10 to 12, wherein the target substance is a receptor present in a cell membrane of a target cell,
the binding substance is an antibody capable of binding to the receptor,
the agent is a medicine that acts on a cell, and
the second substance is a substance produced by action of the medicine on the cell.

15. The information acquisition method according to claim 14, wherein the medicine includes a substance that inhibits DNA replication in the cell, and
the second substance includes a biomarker produced by inhibition of DNA replication in the cell.

16. The information acquisition method according to any one of claims 9 to 15, wherein in the step of obtaining information, a position or an amount of the first substance is acquired as information on the first substance, and a position or an amount of the second substance is acquired as information on the second substance.

17. The information acquisition method according to any one of claims 9 to 16, wherein in the step of obtaining information, information on action or an effect of the first substance is produced based on the information on the first substance and the second substance.

18. The information acquisition method according to claim 10, wherein, in the step of obtaining information, information on a relation between an amount of the first substance or an amount of the binding substance and an amount of the second substance or information on a relation between an amount of the first substance or an amount of the binding substance and a number of a cell in which a second substance is produced is produced.

19. The information acquisition method according to claim 14 or 15, wherein in the step of obtaining information, information on a relation between an amount of the antibody bound to the target cell and an amount of the second substance produced by action of the medicine is obtained as information on action of the first substance.

20. The information acquisition method according to claim 19, wherein the information on action of the first substance is
a number of a cell in which the second substance is produced by action of the medicine, or
a number of a cell that the antibody reached.
